# EUROPÄISCHE PATENTSCHRIFT

(11) **EP 3 629 864 B1**
(45) Veröffentlichungstag und Bekanntmachung des Hinweises auf die Patenterteilung: **14.09.2022**
(21) Anmeldenummer: 18726422.1
(22) Anmeldetag: 17.05.2018
(51) Int. Cl.: A47K 10/18

(54) **HALTESYSTEM ZUM BEREITSTELLEN EINER MIT REINIGUNGS- ODER DESINFEKTIONSTÜCHERN ODER MIT EINMAL-HANDSCHUHEN VERSEHENEN VERPACKUNG**
HOLDING SYSTEM FOR MAKING A PACKAGE AVAILABLE THAT ACCOMMODATES CLEANING OR DISINFECTANT WIPES OR DISPOSABLE GLOVES
SYSTÈME DE RETENUE PRÉVU POUR FOURNIR UN EMBALLAGE MUNI DE LINGETTES DE NETTOYAGE OU DE DÉSINFECTION OU DE GANTS JETABLES

(30) Priorität: 23.05.2017 DE 102017111233
(43) Veröffentlichungstag der Anmeldung: 08.04.2020
(73) Patentinhaber: Bode Chemie GmbH, 22525 Hamburg (DE)
(72) Erfinder: LANGLOTZ, Christian, 20253 Hamburg (DE); KELTING, Matthias, 25554 Bekdorf (DE); KUBOTEIT, Stefan, 21220 Seevetal (DE)
(74) Vertreter: Eibl, Christian
(86) Internationale Anmeldenummer: PCT/EP2018/062890
(87) Internationale Veröffentlichungsnummer: WO 2018/215281

(56) Entgegenhaltungen:
- DE-U1-202010 004 469
- US-A- 3 837 608
- US-A- 5 839 632
- US-A1- 2004 099 623
- US-A1- 2007 290 108
- US-A1- 2016 278 518

## Beschreibung

Die vorliegende Erfindung betrifft ein Haltesystem zum Bereitstellen einer mit Reinigungs-oder Desinfektionstüchern oder mit Einmal-Handschuhen versehenen Verpackung, insbesondere in Gesundheitseinrichtungen.

Professionelle Hygienemaßnahmen sind unumstritten ein Weg zur Verhinderung von Übertragungen mikrobieller Pathogene im Gesundheitswesen, insbesondere in Kranken- oder Pflegeinstitutionen.

Hierbei spielt der Einsatz von Reinigungs- oder Desinfektionstüchern ebenso wie die konsequente Verwendung von Einmal-Handschuhen eine große Rolle. Auch die Art der Ausführung des Reinigungs- oder Desinfektionsvorgangs hat einen entscheidenden Einfluss auf die Effektivität der Reinigung oder Desinfektion.

Daneben kommt aber der Compliance in Bezug auf das Reinigungs- oder Desinfektionsverhalten ebenso wie der Bereitschaft Einmal-Handschuhe zu tragen und zu wechseln eine ebenso herausragende Bedeutung zu, mithin das Verhältnis von einer tatsächlich erfolgten Reinigung oder Desinfektion bzw. Verwendung von Einmal-Handschuhen im Vergleich zu der Anzahl an Gelegenheiten, bei denen eine Reinigung oder Desinfektion bzw. die Verwendung von Einmal-Handschuhe unter hygienischen Gesichtspunkten erfolgen sollte.

Die Gelegenheiten, bei denen eine Reinigung oder Desinfektion erfolgen soll, oder bei denen Einmal-Handschuhe getragen werden sollten, stehen häufig in Zusammenhang mit der Behandlung oder Pflege von Patienten durch das pflegende oder behandelnde Personal.

Es hat sich gezeigt, dass die Compliance verbessert werden kann, wenn die Verfügbarkeit des Reinigungs- oder Desinfektionsmittels bzw. von Einmal-Handschuhen in der Nähe oder besser unmittelbar am sogenannten "Point-of-Care", mithin unmittelbar dort, wo die Behandlung oder Pflege, also der Patientenkontakt, erfolgen soll, gegeben ist.

Es sind daher bereits Haltesysteme bekannt, die die Bereitstellung von Verpackungen für Reinigungstücher, Desinfektionstücher oder Einmal-Handschuhe am oder in der Nähe des Point of Care durch entsprechende Befestigungsmittel ermöglichen.

EP-2915471-A1 beschreibt eine Halterung für eine Tuch-Verpackung. Die Halterung umfasst eine Auflageplatte, seitlich angeordnete Platten und eine rückseitig vorhandene Platte mit einem Befestigungsbereich. DE 20 2010 004469 U1, US 5,839,632, US 2007/290108 A1, US 3,837,608, US 2016/278518 A1 und US 2004/099623 A1 offenbaren andere Haltesysteme. Reinigungstücher, Desinfektionstücher und Einmal-Handschuhe werden üblicherweise in Folienbeuteln oder in Verpackungen aus einem Pappmaterial bereitgestellt, wobei die Verpackung eine gegebenenfalls mit einem Deckel verschließbare Entnahmeöffnung aufweist.

Reinigungstücher oder Desinfektionstücher liegen in der Verpackung häufig als gefalteter Stapel vor, wobei die Faltung so erfolgt, dass beim Herausziehen eines aus der Entnahmeöffnung herausragenden Tuches das nachfolgende, weiter unten im Stapel vorhandene Tuch partiell mitgenommen wird, so dass dieses nun in der Entnahmeöffnung für einen Nutzer greifbar bereitgestellt wird. Die Ablösung des herausgezogenen Tuches von dem nachfolgenden Tuch wird durch die Reibung des nachfolgenden Tuches an der Innenseite der Verpackung unterstützt. Ein ähnlicher Effekt erleichtert einem Nutzer auch das Herausnehmen von Einmal-Handschuhen aus einer Verpackung, wobei in diesem Falle die Handschuhe aufgrund von Adhäsion trennbar aneinanderhaften.

Bei der Entnahme von Tüchern oder Handschuhen aus der Verpackung kann sich das Problem ergeben, dass der bei einer zunehmenden Entleerung der Verpackung entstehende Hohlraum zwischen den verbleibenden Handschuhen und der Entnahmeöffnung das weitere Herausnehmen des Artikels erschwert. Zum einen lässt nämlich die oben geschilderte Friktion zwischen dem Artikel und der Verpackung nach, da bei einem kleiner werdenden Füllstand die von den Artikeln auf die Verpackung ausgeübte Spannung zurückgeht. Dies kann dazu führen, dass der Nutzer beim Herausziehen des in der Entnahmeöffnung vorhandenen Artikels eine Vielzahl weiterer Artikel nachzieht, da sich diese nicht wie vorgesehen voneinander lösen. Zum anderen muss der Nutzer, wenn sich einmal kein Artikel greifbar in der Entnahmeöffnung befindet, tiefer in die Verpackung hineingreifen, um den Artikel, also das Reinigungstuch, das Desinfektionstuch oder den Einmal-Handschuh, herausnehmen zu können.

EP-2974980-A1 offenbart einen Tuchspender, bei dem die Verpackung ein im Bereich der Entnahmeöffnung vorhandenes und nach innen drückendes Element aufweist. Das Element kann einen Druck auf die Oberseite des Tücherstapels ausüben, um die bei einer zunehmenden Entleerung der Verpackung reduzierte Spannung des Stapels auf die Innenseite der Verpackung zu kompensieren.

Bei einer horizontalen Bereitstellung einer Verpackung mit feuchten Reinigungstüchern oder Desinfektionstüchern am "Point-of-care" (also mit einer nach oben zeigenden Entnahmeöffnung) ergibt sich häufig das weitere Problem, dass die in der Verpackung vorhandene Flüssigkeit zum Boden der Verpackung sickert, mit der Folge, dass die weiter oben im Stapel vorhandenen Tücher nicht ausreichend mit der Reinigungs-oder Desinfektionslösung getränkt sind. Um diesem Problem zu begegnen, wäre es vorteilhaft, die Verpackung am "Point-of-care" in einer vertikalen Position bereitzustellen (also mit einer zur Seite hinzeigenden Entnahmeöffnung). Anderseits kann es gerade im Falle der Bereitstellung von Einmal-Handschuhen am "Point-of-care" vorteilhaft sein, diese in einer horizontal gelagerten Verpackung zu präsentieren, da sich dann die Entnahme einfacher gestaltet. Im Stand der Technik bekannte Halterungen erlauben jedoch keine wahlweise Bereitstellung von Verpackungen in horizontaler oder vertikaler Ausrichtung.

Die bekannten Lösungen haben sich zudem hinsichtlich ihrer Positionierbarkeit am "Point-of-care" als unzureichend erwiesen.

Im Sinne der Compliance ist es weiterhin wünschenswert, dass die Verpackung mühelos in das Haltesystem eingebracht werden kann und danach in einer Anwendungssituation sicher gehalten wird.

Die Aufgabe der vorliegenden Erfindung ist daher, die Handhabung bekannter Haltesysteme zu verbessern. Insbesondere soll eine Verbesserung hinsichtlich der Befestigung der Verpackung am Haltesystem sowie eine verbesserte Positionierbarkeit und Flexibilität des Haltesystems erreicht werden.

Diese Aufgabe wird gelöst durch ein Haltesystem nach Anspruch 1 zum Bereitstellen einer insbesondere mit Reinigungs- oder Desinfektionstüchern oder mit Einmal-Handschuhen versehenen Verpackung, wobei die Verpackung eine Entnahmeöffnung aufweist, umfassend:
- einen Halter mit einem Aufnahmebereich für die Verpackung, wobei der Halter einen ersten Befestigungsbereich, einen zweiten Befestigungsbereich und einen dritten Befestigungsbereich aufweist, wobei der erste, zweite und dritte Befestigungsbereich Drähte oder Stäbe umfassen,
- ein Befestigungsmittel zur lösbaren Befestigung des Halters an Gegenständen, wobei das Befestigungsmittel an dem ersten Befestigungsbereich fixierbar ist, und das Befestigungsmittel dadurch eine erste Halteposition einnehmen kann, und das Befestigungsmittel an dem zweiten Befestigungsbereich fixierbar ist, und das Befestigungsmittel dadurch eine zweite Halteposition einnehmen kann, und das Befestigungsmittel (7) an dem dritten Befestigungsbereich fixierbar ist, und das Befestigungsmittel dadurch eine dritte Halteposition einnehmen kann,
- und ein an dem Halter vorhandenes Federelement, welches eine Kraft auf die in den Aufnahmebereich eingelegte Verpackung ausüben kann, so dass die Verpackung in dem Aufnahmebereich gehalten wird.

Mit der Formulierung "fixierbar" und "eine Halteposition einnehmen kann" wird zum Ausdruck gebracht, dass die erste Halteposition, die zweite Halteposition und die dritte Halteposition Alternativen darstellen, die sich jeweils durch eine Befestigung des Befestigungsmittels an dem ersten Befestigungsbereich oder an dem zweiten Befestigungsbereich oder an dem dritten Befestigungsbereich verwirklichen lassen.

Das erfindungsgemäße Haltesystem erlaubt dem Anwender eine flexible Positionierung von Verpackungen unmittelbar am "Point-of-care". Das Haltesystem kann an unterschiedlichsten Gegenständen fixiert werden, beispielsweise an vertikal, schräg oder horizontal orientierten Stäben ebenso wie an Platten. Weiterhin hat es sich gegenüber aus dem Stand der Technik bekannten Haltern als vorteilhaft erwiesen, dass die Verpackungen mit nur einer einzigen Hand in das Haltesystem eingebracht und entnommen werden können ("Einhandbedienbarkeit"). Auch die in der Verpackung enthaltenen Artikel können aus der in dem Haltesystem sicher gehaltenen Packung problemlos mit einer einzigen Hand entnommen werden.

Die Reinigungs- oder Desinfektionstücher können mit einem insbesondere flüssigem, gelartigem, pastösen oder schaumförmigen oder schäumbaren Mittel beaufschlagt sein. Die Desinfektionstücher können insbesondere mit einem alkoholischen und nichtalkoholischen Desinfektionsmittel getränkt sein. Die Reinigungstücher können insbesondere mit flüssigen Seifen oder Reinigungslösungen getränkt sein. Die Einmal-Handschuhe können beispielsweise aus Latex-, aus Nitril-, aus Vinyl- oder aus Polyethylen-Materialien gefertigt sein.

Die Form der Verpackung kann vorzugsweise im Wesentlichen quaderförmig ausgebildet sein. Vorzugsweise weist die Verpackung eine Höhe H auf, deren Erstreckung kleiner, insbesondere mindestens um den Faktor 1,5 kleiner ist als die Erstreckung der Breite B der Verpackung. Vorzugsweise ist die Entnahmeöffnung der Verpackung mittig an einer Seitenfläche, insbesondere an einer Deckfläche, vorhanden. Denkbar und vorteilhaft ist weiterhin, die Verpackung beutelartig auszubilden. Weiter vorzugsweise kann die Verpackung als Bag-in Box - Verbundbehälter ausgebildet sein. Hierbei ist ein innerer Beutel innerhalb einer im Vergleich zum Beutel aus steiferem Material gebildeten äußeren Box aus beispielsweise Kunststoff oder Karton aufgenommen. Des Weiteren ist bevorzugt, dass es sich bei der Verpackung um eine Flowpack-Verpackung handelt, welche insbesondere ein Verschlusselement aufweist, welches geeignet ist, die Entnahmeöffnung zu verschließen.

Hinsichtlich der Haltepositionen ist es bevorzugt, dass der Aufnahmebereich des Halters derart ausrichtbar ist, dass die Verpackung in einer Anwendungssituation im Wesentlichen auf einer Seite liegend in dem Aufnahmebereich aufgenommen ist, um dem Nutzer in einer Anwendungssituation einen uneingeschränkten Zugang zu der Entnahmeöffnung zu gewährleisten.

Die mindestens erste, zweite und dritte Halteposition ermöglichen dem Anwender, das Befestigungsmittel unterschiedlich auszurichten, um den Halter wahlweise an unterschiedlich sich im Raum erstreckenden und angeordneten Gegenständen, wie vertikal oder horizontal oder schräg verlaufenden Platten, Stangen oder Regalen, oder Bauteilen von Tischen, Stühlen, Bettkanten oder Bettstangen sowie Infusionsständern, Stationswagen, Servierwagen oder Ähnlichem zu befestigen.

Hierfür kann der Anwender je nach Vorhandensein geeigneter Gegenstände das Befestigungsmittel an dem ersten oder zweiten oder dritten Befestigungsbereich fixieren.

Nach einer bevorzugten Ausführungsform ist vorgesehen, dass die erste Halteposition die Befestigung des Halters an einem horizontal orientierten Gegenstand ermöglicht und die zweite Halteposition die Befestigung des Halters an einem vertikal orientierten Gegenstand ermöglicht und die dritte Halteposition die Befestigung des Halters an einem schräg orientierten Gegenstand ermöglicht.

Dabei bedeutet "horizontal" parallel zu einer Ebene, welche parallel zum Horizont verläuft, das heißt senkrecht zur Lotrichtung. "Vertikal" bedeutet parallel zu einer Ebene, die parallel zur Lotrichtung verläuft. "Schräg" bedeutet parallel zu einer Ebene, welche sowohl eine horizontale als auch eine vertikale Ebene schneidet.

Die dritte Halteposition ermöglicht vorzugsweise die Befestigung des Halters an einem zwischen 10° und 80°, insbesondere zwischen 20° und 60°, weiter insbesondere zwischen 30° und 50° zur Horizontalen geneigten Gegenstand.

Das Befestigungsmittel kann in an sich beliebiger Weise, insbesondere aber formschlüssig und/oder kraftschlüssig an dem ersten als auch an dem zweiten, als auch an dem dritten Befestigungsbereich fixierbar sein.

Vorteilhafterweise bilden die Drähte oder Stäbe des ersten, zweiten oder dritten Befestigungsbereichs gleichzeitig Abschnitte des Aufnahmebereichs des Halters.

Hierbei bilden die Drähte oder Stäbe des ersten Befestigungsbereichs mindestens einen Abschnitt eines Bodens des Aufnahmebereichs.

Die Drähte oder Stäbe oder plattenförmigen Bauteile des zweiten Befestigungsbereichs bilden mindestens einen Abschnitt eines Rückteils des Aufnahmebereichs.

Wenn vorstehend oder im Folgenden die Pluralform "Drähte oder Stäbe" benutzt wird, so soll die Singularform "der Draht oder der Stab" hiermit ebenfalls erfasst sein.

Der dritte Befestigungsbereich ist vorzugsweise zwischen dem ersten und zweiten Befestigungsbereich angeordnet.

Der Aufnahmebereich weist vorzugsweise vordere Stützelemente auf, die vorzugsweise durch Drähte oder Stäbe gebildet sind und welche weiter bevorzugt einen Abschnitt eines Vorderteils des Aufnahmebereichs bilden. Die vorderen Stützelemente sind allerdings vorzugsweise nicht dafür eingerichtet, das Befestigungsmittel daran fixieren zu können.

Der Aufnahmebereich ist vorzugsweise als ein nach oben offenes Gestell ausgebildet, so dass eine Verpackung ohne Werkzeug und ohne Schließ- oder Feststellvorrichtungen zu betätigen gegen den Widerstand eines vorzugsweise zur Rückseite hin ausweichenden Federelementes von oben in das Gestell platziert und wieder entnommen werden kann. Beim Einlegen der Verpackung in den Aufnahmebereich schiebt der Nutzer demnach die Verpackung gegen den Wiederstand des an dem Halter vorhandenen Federelementes in das Haltesystem. Eine sichere Fixierung der Verpackung im Aufnahmebereich wird dann durch das Federelement gewährleistet.

Der erste Befestigungsbereich weist erfindungsgemäß Drähte oder Stäbe auf oder ist vorzugsweise daraus gebildet, das heißt er besteht vorzugsweise daraus. Insbesondere verlaufen diese Drähte oder Stäbe in einer Anwendungssituation parallel zur Horizontalen, also parallel zu einer Ebene, welche parallel zum Horizont verläuft. Als eine Anwendungssituation wird im Rahmen der vorliegenden Erfindung eine Situation verstanden, bei der eine Verpackung für den Anwender in den Aufnahmebereich des Halters aufnehmbar ist oder bereits in dem Aufnahmebereich platziert ist.

Der zweite Befestigungsbereich weist erfindungsgemäß Drähte oder Stäbe auf oder ist vorzugsweise daraus gebildet. Insbesondere verlaufen diese Drähte oder Stäbe senkrecht zu den Drähten oder Stäben des ersten Befestigungsbereichs.

Der dritte Befestigungsbereich weist ebenfalls Drähte oder Stäbe auf oder ist daraus gebildet. Diese Drähte oder Stäbe des dritten Befestigungsbereichs verlaufen schräg, insbesondere in einem Winkel von 10° bis 80°, weiter insbesondere von 20° bis 60°, weiter insbesondere von 30 bis 50° zu den Drähten oder Stäben des ersten Befestigungsbereichs.

Die Drähte oder Stäbe sind insbesondere aus an sich bekannten Materialien wie Kunststoffen oder Metallen, insbesondere Aluminium oder Stahl, gebildet. Vorzugsweise handelt es sich um gegenüber alkoholischen Lösungen unempfindliche Materialien, um den Halter regelmäßig reinigen und desinfizieren zu können.

Weiter vorzugsweise handelt es sich um Materialien, die sterilisierbar, insbesondere dampfsterilisierbar, sind.

Die Drähte oder Stäbe können massiv oder hohl, das heißt röhrenförmig, ausgebildet sein und sie weisen vorzugsweise einen runden, insbesondere kreisförmigen Querschnitt auf. Solchenfalls werden die Stäbe nachfolgend auch als Rundstäbe bezeichnet. Der Durchmesser der Stäbe beträgt insbesondere 1-10 mm, weiter insbesondere 3-6 mm. Andere, insbesondere drei- vier-, oder mehreckige Querschnittsformen sind ebenfalls denkbar.

Nach weiteren bevorzugten Ausführungsformen umfasst der erste oder der zweite oder der dritte Befestigungsbereich jeweils ein Paar parallel verlaufender Drähte oder Stäbe ist daraus gebildet.

Vorzugsweise verläuft ein Paar parallel verlaufender Drähte oder Stäbe des ersten Befestigungsbereichs in einer ersten Ebene und ein Paar parallel verlaufender Drähte oder Stäbe des zweiten Befestigungsbereichs verläuft in einer zweiten Ebene und ein Paar parallel verlaufender Drähte oder Stäbe des dritten Befestigungsbereichs verläuft in einer dritten Ebene, wobei keine der ersten bis dritten Ebenen zu einer anderen der ersten bis dritten Ebenen parallel verläuft.

Ein jeweiliges Paar parallel verlaufender Drähte oder Stäbe ist vorzugsweise jeweils äquidistant angeordnet. Das bedeutet, dass die Abstände zwischen zwei ein jeweiliges Paar bildenden Drähten oder Stäben des ersten Befestigungsbereichs und des zweiten Befestigungsbereichs und des dritten Befestigungsbereichs identisch sind. Diese Ausführungsform ermöglicht, wie weiter unten näher erläutert wird, einfache und benutzerfreundliche Varianten zur flexiblen Fixierung des Befestigungsmittels an dem Halter. Nach einer weiteren bevorzugten Ausführungsform umfassen auch die vorderen Stützelemente jeweils ein Paar parallel verlaufender Drähte oder Stäbe oder sind daraus gebildet.

Nach einer besonders bevorzugten Ausführungsform ist der Halter inklusive seines Aufnahmebereichs und seiner Befestigungsbereiche durch einen durchgehenden Draht oder Stab gebildet. Hierbei kann der durchgehende Draht oder Stab aus zwei oder mehreren aneinandergefügten Abschnitten gebildet sein. Hierbei kommen an sich bekannte Fügeverfahren, je nach dem verwendeten Material in Frage, wie beispielsweise stoffschlüssige Fügeverfahren wie Kleben, Schweißen oder Löten. Denkbar sind des Weiteren Steckverbindungen oder andere form- und/oder kraftschlüssige Verbindungstechniken.

Ganz besonders bevorzugt ist der Draht oder Stab jedoch aus einem einzigen, insbesondere abschnittsweise gebogenen oder gekrümmt geformten Draht oder Stab gebildet.

Das Befestigungsmittel weist vorzugsweise eine lösbare Klemmvorrichtung auf, vermittels derer die Befestigung des Halters an den Gegenständen herstellbar ist. Andere an sich bekannte Befestigungsmittel, wie aus dem Zweiradbereich bekannte, insbesondere manuell und weiter insbesondere ohne weiteres Werkzeug bedienbare Schnellverschlüsse sind denkbar und vom Erfindungsgedanken umfasst.

Die Klemmvorrichtung umfasst vorzugsweise eine erste Klemmbacke und eine zweite Klemmbacke, wobei der Abstand der ersten Klemmbacke von der zweiten Klemmbacke veränderbar ist. Vorzugsweise sind die wirksamen Klemmbereiche der Klemmbacken, insbesondere hinsichtlich ihrer Form unterschiedlich ausgebildet. So kann der wirksame Klemmbereich der ersten Klemmbacke an die Form eines flachen, ebenen Gegenstandes wie eine Tisch- oder Bettkante angepasst sein. Vorzugsweise ist der wirksame Klemmbereich der zweiten Klemmbacke mit einer V- oder U-förmigen Ausbuchtung versehen und damit angenähert oder angepasst an die Form eines Rohres oder einer Stange oder eines Stabes eines Bettgestells oder eines Stativs oder eines anderen stangen- oder stabartigen Gegenstandes.

Des Weiteren ist es vorteilhaft, die wirksamen Klemmbereiche der ersten oder zweiten Klemmbacke mit einer rutschhemmenden und/oder die Oberfläche der Gegenstände schonenden Beschichtung, insbesondere aus PVC, Latex, Silikon oder vulkanisiertem Gummi, zu versehen.

Die Klemmbacken sind vorzugsweise durch eine insbesondere manuell betätigbare Vorrichtung, insbesondere eine Schraubvorrichtung, aufeinander zu und voneinander wegbewegbar.

Insbesondere kann es vorteilhaft sein, dass die Klemmbacken gegen eine Federkraft aufeinander zu bewegbar sind.

Nach einer bevorzugten Ausführungsform weist das Befestigungsmittel eine Bediensperre auf. Die Bediensperre ist insbesondere geeignet, das Befestigungsmittel gegen unbefugten Gebrauch oder das Haltesystem in Gänze vor Entwendung zu schützen. Vorzugsweise weist das Befestigungsmittel hierzu eine Schließvorrichtung auf, die in verschlossenem Zustand verhindert, dass das Befestigungsmittel bedienbar ist. Vorzugsweise handelt es sich hierbei um an sich, beispielsweise aus dem Bereich der für die Montage an ein Kraftfahrzeug geeigneten Fahrradträger bekannte, mittels eines Schlüssels oder Eingabe einer Zahlen-oder Ziffern- oder Zeichenkombination offen- und schließbare Schließvorrichtungen. Die Bediensperre kann nach einer alternativen Ausführungsform auch dadurch gebildet sein, dass das Befestigungsmittel lediglich durch ein Spezialwerkzeug wie beispielsweise einen speziell ausgestalteten Innenkantschlüssel, wie einen Innenfünfkantschlüssel, bedienbar ist.

Die Bedienung des Befestigungsmittels hat sich als besonders komfortabel erwiesen, wenn vermittels des Befestigungsmittels, insbesondere der Betätigung der Schraubvorrichtung, sowohl eine Befestigung des Halters an den Gegenständen, als auch gleichzeitig eine insbesondere kraftschlüssige Fixierung des Befestigungsmittels an den ersten oder an den zweiten oder an den dritten Befestigungsbereichen herstellbar ist.

Das Befestigungsmittel weist vorzugsweise ein Anschlussmittel auf, vermittels dessen das Befestigungsmittel an dem Halter insbesondere anschließbar, oder weiter insbesondere gehalten und fixierbar ist. Des Weiteren ist das Anschlussmittel vorzugsweise dafür eingerichtet, das Befestigungsmittel insbesondere manuell zwischen jeder der ersten bis dritten Haltepositionen bewegen, insbesondere verschieben zu können.

Nach einer besonders bevorzugten Ausführungsform der Erfindung ist das Befestigungsmittel unverlierbar an dem Halter, insbesondere formschlüssig unverlierbar gehalten. Das kann vorzugsweise bereits herstellerseitig vorgesehen sein.

Es hat sich als besonders vorteilhaft erwiesen, wenn der Halter relativ zu dem Befestigungsmittel, das heißt zumindest zu einem der Bauteile des Befestigungsmittels, um eine Achse um vorzugsweise bis zu 360° drehbar, insbesondere stufenlos drehbar gelagert ist. Hierdurch kann der Halter in einer der Haltepositionen durch Rotation in für die Darreichung geeignete Endposition gebracht werden.

Nach einer weiteren bevorzugten Ausführungsform des Haltesystems ist eine Befestigungsbereichsebene, also die Ebene, in welcher das Paar die jeweiligen Befestigungsbereiche des Halters bildenden Rundstäbe in einer der Haltepositionen verlaufen, parallel zu einer Gegenstandsebene orientiert, also zu derjenigen Ebene parallel orientiert, in der die Gegenstände verlaufen, an welche in einer jeweiligen Halteposition die Klemmvorrichtung befestigbar ist.

Wie vorstehend bereits erläutert ist eine Verpackung in einer Halteposition im Wesentlichen seitlich liegend in dem Aufnahmebereich des Halters aufgenommen. Dies hat sich für die Anwendungssituation als eine besonders geeignete Position erwiesen. Hierdurch ist sichergestellt, dass die Entnahmeöffnung zugänglich ist.

An dem Halter ist ein Federelement vorhanden, welches eine Kraft auf die in den Aufnahmebereich eingelegte Verpackung ausüben kann, so dass die Verpackung in dem Aufnahmebereich gehalten wird. Das Federelement ragt in einem ungespannten Zustand in den Aufnahmebereich hinein und verengt diesen. Beim Einlegen der Verpackung in den Aufnahmebereich wird das Federelement aus dem Aufnahmebereich wegbewegt und geht hierdurch in einen gespannten Zustand über, wobei gleichzeitig eine Federkraft auf die Verpackung ausgeübt wird. Hierdurch wird die Verpackung sicher in dem Aufnahmebereich fixiert und kann gleichzeitig durch einen Nutzer mühelos wieder herausgezogen werden. Vorzugsweise ist das Federelement derart an dem Halter angebracht, dass eine Kraft auf die der Entnahmeöffnung gegenüberliegende Seite der in den Aufnahmebereich eingelegten Verpackung ausgeübt werden kann. Dies lässt sich am besten durch eine Befestigung des Federelements am Rückteil des Halters, also an den Drähten oder Stäben des dritten Befestigungsbereichs bewerkstelligen. Vorzugsweise umfasst das Federelement eine Befestigungskomponente zum Anbringen des Federelements am dritten Befestigungsbereich sowie eine Federkomponente, welche in den Aufnahmebereich hineinragt. Befestigungskomponente und Federkomponente können einstückig ausgebildet sein. Es handelt sich bei der Federkomponente um eine Blattfeder, welche einstückig mit der Befestigungskomponente ausgebildet sein kann oder welche an der Befestigungskomponente unlösbar angebracht ist. Die Blattfeder sollte möglichst im oberen Bereich des dritten Befestigungsbereichs mit der Befestigungskomponente verbunden sein und schräg nach unten in den Aufnahmebereich hineinragen. Eine derartige Anordnung gewährleistet ein Einschieben der Verpackung in den Aufnahmebereich gegen den zunehmenden Widerstand der Federkomponente. Das offene Ende der Blattfeder soll vorzugsweise mittig gegen eine in den Aufnahmebereich eingebrachte Verpackung drücken. Dies hat den Vorteil, dass die in der vorzugsweise flexibel ausgebildeten Verpackung vorhandenen Artikel zur Aufnahmeöffnung hingedrückt werden, wodurch eine Entnahme der Artikel aus der Entnahmeöffnung erleichtert wird.

Eine weitere bevorzugte Ausführungsform betrifft ein System umfassend
- ein Haltesystem nach einer oder mehrerer der vor- oder nachstehend beschriebenen Varianten und
- eine mit Reinigungs- oder Desinfektionstüchern oder mit Einmal-Handschuhen versehene Verpackung,
wobei die Verpackung in den Aufnahmebereich einbringbar ist.

Vorzugsweise ist die Verpackung zur einmaligen Verwendung vorgesehen, während das Haltesystem zur Wiederverwendung geeignet ist.

In besonders geeigneter Weise ist die Verpackung aus einem flexiblen Material, insbesondere Kunststoff oder Pappe, gebildet. Vorteilhaft lässt sich die der Entnahmeöffnung gegenüberliegende Unterseite der Verpackung und gegebenenfalls die Oberseite der Verpackung durch leichten Druck, beispielsweise leichten manuellen Druck, eindrücken, wobei die Wände der Verpackung nach dem Eindrücken unmittelbar in ihre ursprüngliche Form zurückfedert. Hierdurch kann die Verpackung in besonders vorteilhafter Weise kraft- und formschlüssig in den Aufnahmebereich des Halters aufnehmbar sein. Weiterhin bewirkt ein Eindrücken der Unterseite der Verpackung durch das Federelement ein Verschieben des Verpackungsinhaltes auf die Entnahmeöffnung zu, so dass der in der Verpackung enthaltene Artikel besonders einfach entnehmbar ist, da er sich näher an der Entnahmeöffnung befindet. Das Eindrücken der Unterseite der Verpackung durch das Federelement führt zudem zu einer Verringerung des Hohlraumes, welcher bei einer zunehmenden Entleerung der Verpackung entsteht. Hierdurch bleibt eine Friktion des Artikels mit der Verpackungsinnenseite erhalten, welche ein Ablösen eines entnommenen Artikels von weiteren noch in der Verpackung vorhandenen Artikeln erleichtert.

Weiter bevorzugt weist der Aufnahmebereich des Halters eine Tiefe FT auf, welche größer ist, insbesondere um 5-50 mm, weiter insbesondere um 10-30 mm größer ist als die Höhe h der Verpackung.

In besonders vorteilhafter Weise ist der Halter so ausgebildet, dass die Deckfläche einer in den Aufnahmebereich aufgenommenen Verpackung weitestgehend sichtbar ist, so dass die Deckfläche mit für den Anwender wichtigen Informationen versehen werden kann, ohne dass diese durch den Halter verdeckt würde.

Im Falle einer im Wesentlichen quaderförmig ausgebildeten Verpackung mit entsprechend vier Seitenflächen sind vorzugsweise mindestens drei der Seitenflächen zu mindestens 90%, insbesondere zu mindestens 95%, weiter insbesondere zu mindestens 97% der jeweiligen Fläche durch den Anwender frei betracht- und einsehbar.

Eine weitere Ausführungsform betrifft eine Anordnung umfassend mindestens zwei Haltesysteme nach einer oder mehrerer der vor- oder nachstehend beschriebenen Varianten und vorzugsweise mindestens erste und zweite Verpackungen der vor- oder nachstehend beschriebenen Varianten, wobei ein erstes Haltesystem einen ersten Halter mit einem ersten Aufnahmebereich aufweist und wobei ein zweites Haltesystem einen zweiten Halter mit einem zweiten Aufnahmebereich aufweist und wobei sich der erste Aufnahmebereich von dem zweiten Aufnahmebereich unterscheidet. Vorzugsweise unterscheidet sich der erste Aufnahmebereich von dem zweiten Aufnahmebereich derart, dass der erste Aufnahmebereich eine im Vergleich zum zweiten Aufnahmebereich hinsichtlich der äußeren Form und/oder der Abmessungen und/oder des maximal möglichen Inhalts, gemessen in Millilitern, unterschiedliche Verpackungen aufnehmen kann.

Vorzugsweise weist der erste Aufnahmebereich eine Tiefe FT, eine Höhe FH und eine Breite FB auf, und der zweite Aufnahmebereich weist eine Tiefe FT, eine Höhe FH und eine Breite FB auf, wobei sich der erste Aufnahmebereich von dem zweiten Aufnahmebereich durch mindestens eine Abmessung ausgewählt aus der Höhe FH, Tiefe FT, Breite FB unterscheidet.

Im Falle, dass der erste oder der zweite oder der dritte Befestigungsbereich des ersten und zweiten Haltesystems jeweils ein Paar parallel verlaufender Drähte oder Stäbe aufweisen oder daraus gebildet sind, sind ein jeweiliges Paar parallel verlaufender Drähte oder Stäbe vorzugsweise äquidistant angeordnet. Das bedeutet, dass diese jeweils ein Paar bildende Drähte oder Stäbe des ersten Haltesystems denselben Abstand voneinander aufweisen wie die ein Paar bildenden Drähte oder Stäbe des zweiten Haltesystems. Diese Ausführungsform ermöglicht das erste und zweite Haltesystem mit einem zumindest hinsichtlich seines Anschlussmittels identischen Befestigungsmittel auszustatten.

In besonders vorteilhafter Weise umfasst das erste Haltesystem mithin ein erstes Befestigungsmittel und das zweite Haltesystem umfasst ein zweites Befestigungsmittel, wobei die Anschlussmittel des ersten und zweiten Befestigungsmittels identisch ausgebildet sind. Insbesondere sind die ersten und zweiten Befestigungsmittel gänzlich identisch ausgebildet.

Nach einer weiteren Variante der Erfindung weist die erste Verpackung eine Länge I, eine Höhe h und eine Breite b auf und die zweite Verpackung weist eine Länge I, eine Höhe h und eine Breite b auf, wobei sich die erste Verpackung von der zweiten Verpackung durch mindestens eine Abmessung ausgewählt aus der Höhe h, Länge I, Breite b unterscheidet.

In den Figuren zeigen
Figur 1 ein erfindungsgemäßes Haltesystem
Figur 2 den Halter der Figur 1 in einer weiteren Ansicht
Figuren 3a-3c Ansichten eines erfindungsgemäßen Haltesystems in erster, zweiter und dritter Halteposition
Figur 4a eine Teilansicht einer Schnittebene durch ein erfindungsgemäßes Haltesystem
Figur 4b eine der Figur 4a entsprechende Ansicht, jedoch mit von der Federkraft einer Spiralfeder befreitem Anschlussmittel
Figuren 5a und 5b schematische Ansichten eines an einem Gegenstand befestigten Haltesystems
Figur 6 eine schematische Ansicht einer in das Haltesystem aufgenommenen Verpackung
Figur 7a-7b Bemaßung des Halters sowie eine Ansicht einer in ein Haltesystem aufgenommenen Verpackung
Figur 8 einen weiteren Halter

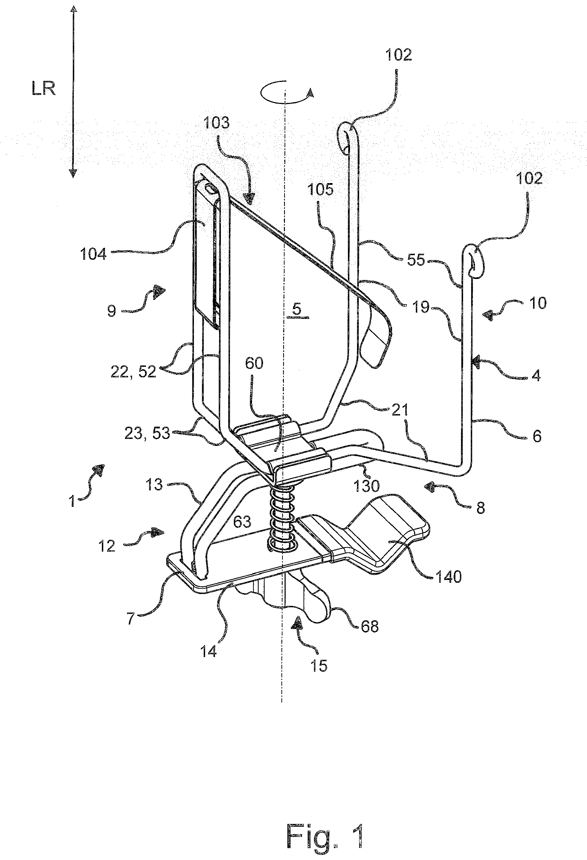

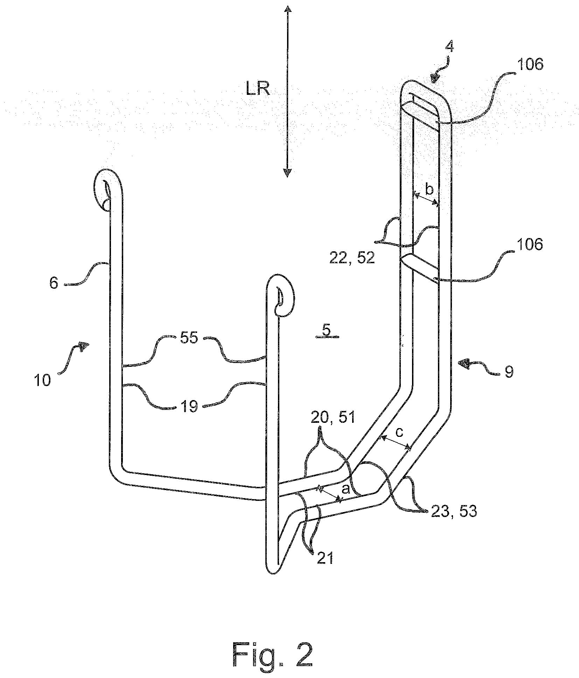

Figur 1 zeigt eine Ausführungsform eines erfindungsgemäßen Haltesystems 1. In Figur 2 ist der Halter in einer weiteren Ansicht (um 180° gedreht) jedoch ohne herstellerseitig versehenes Befestigungsmittel dargestellt. Die nachfolgende Beschreibung erfolgt zunächst unter Bezugnahme auf Figuren 1 und 2. Das Haltesystem 1 weist einen Halter 4 und ein

Befestigungsmittel 7 auf. Der Halter 4 besitzt einen Aufnahmebereich 5, welcher dafür eingerichtet ist, eine nicht dargestellte Verpackung aufzunehmen. Hierfür ist der Halter 4 aus mehreren Abschnitten eines einzigen durchgehenden, abschnittsweise gebogenen und den Aufnahmebereich 5 bildenden Rundstabs 6 gebildet. Die beiden Enden 102 des Rundstabes sind ösenförmig abgebogen. Der Rundstab 6 besteht aus Edelstahl und weist einen Durchmesser von 4,0 mm auf. Wenn im Folgenden der Begriff "Rundstäbe" oder "Rundstab" benutzt wird sind hiermit auch Abschnitte des durchgehenden Rundstabes 6 gemeint.

Der Aufnahmebereich 5 umfasst einen Boden 8, ein Rückteil 9 und ein Vorderteil 10. Das Vorderteil 10 weist ein Paar parallel in Längsrichtung LR des Halters 4 verlaufender Rundstäbe 19 zur Bildung von vorderen Stützelementen 55 einer Länge von 80 mm auf, welche einen Abstand voneinander von 80 mm oder nach einer zweiten Ausführungsform des Halters 4 von 65 mm aufweisen.

Der Boden 8 weist in einem ersten Abschnitt ein Paar quer (90°) zu der Längsrichtung LR und parallel zueinander verlaufender Rundstäbe 20 einer Länge von 20 mm auf, die in einer ersten Ebene verlaufen (in Figur 2 dargestellt). Der Abstand dieser Rundstäbe 20 in diesem ersten Abschnitt des Bodens 8 voneinander beträgt a=20 mm. Wie weiter unten näher erläutert bilden diese Rundstäbe einen ersten Befestigungsbereich 51.

In einem zweiten Abschnitt des Bodens 8 gehen die Rundstäbe 20 des ersten Abschnitts des Bodens 8 in zwei um 45° nach außen abknickende Rundstäbe 21 einer Länge von 25 mm oder nach einer zweiten Ausführungsform des Halters 4 einer Länge von 40 mm über, deren Abstand voneinander sich mit Entfernung von dem ersten Abschnitt des Bodens 8 stetig vergrößert, wobei die Rundstäbe 21 des zweiten Abschnitts des Bodens 8 ebenfalls in der ersten Ebene verlaufen, um schließlich in einem kurzen Bogen in die im Wesentlichen in Längsrichtung LR des Halters 4 verlaufenden Rundstäbe 19 des Vorderteils 10 überzugehen.

Das Rückteil 9 weist in einem ersten Abschnitt ein Paar parallel in Längsrichtung LR des Halters und damit senkrecht zu den Rundstäben 20 des ersten Abschnitts des Bodens und in einer zweiten Ebene verlaufender Rundstäbe 22 einer Länge von 40 mm auf, welche einen Abstand voneinander von b=20 mm aufweisen. Wie weiter unten näher erläutert bilden diese Rundstäbe 22 einen zweiten Befestigungsbereich 52.

Zwischen diesen Rundstäben 22 des Rückteils 9 und den Rundstäben 20 des ersten Abschnitts des Bodens 8 ist in einem Zwischenteil des Halters 4 ein weiteres Paar parallel verlaufender Rundstäbe 23 schräg und zwar in einem Winkel von 45° zu den Rundstäben 20 des ersten Abschnitts des Bodens 8 und in einer dritten Ebene verlaufend angeordnet, derart dass dieses weitere Paar parallel verlaufender Rundstäbe 23, welche eine Länge von 28 mm aufweisen, die Rundstäbe 20 des ersten Abschnitts des Bodens 8 mit denen des ersten Abschnitts des Rückteils verbindet, die jeweiligen Rundstäbe gehen also jeweils in einem kurzen Bogen ineinander über. Auch der Abstand der Rundstäbe 23 in dem Zwischenteil beträgt c= 20 mm. Wie weiter unten näher erläutert bilden diese Rundstäbe einen dritten Befestigungsbereich 53.

Im oberen Bereich des Rückteils 9 ist das Federelement 103 befestigt. Bei der in den Figuren 1, 3, 5, 6, 7 und 8 gezeigten Ausführungsform des Federelements 103 umfasst das Federelement die Befestigungskomponente 104 und die Federkomponente 105. Die Befestigungskomponente 104 ist an zwei Streben 106, welche zwischen den Rundstäben 22 vorhanden sind, am Rückteil 9 des Halters angebracht (siehe auch Figur 2). Die Federkomponente 105 ist als Blattfeder 105 ausgeführt, welche sich schräg nach unten in den Aufnahmebereich 5 erstreckt. Die Blattfeder 105 wird beim Einlegen einer Verpackung 2 in den Aufnahmebereich 5 zum Rückteil 9 des Halters hin weggedrückt.

Der Halter (Figur 2) weist vorliegend drei durch jeweils ein Paar parallel verlaufender Rundstäbe 20, 22, 23 gebildeter Befestigungsbereiche 51, 52, 53 auf, an denen das Befestigungsmittel 7 fixiert werden kann. In Figur 1 ist das Befestigungsmittel an dem ersten Befestigungsbereich fixiert, so dass das weiter unten noch näher beschriebene Anschlussmittel 60 die Sicht auf die den ersten Befestigungsbereich bildenden Rundstäbe in Figur 1 verdeckt.

Das in Figur 1 dargestellte Befestigungsmittel 7 weist eine Klemmvorrichtung 12 auf, vermittels derer die Befestigung des Halters 4 an den Gegenständen erfolgen kann. Die Klemmvorrichtung 12 weist eine erste Klemmbacke 13 und eine zweite Klemmbacke 14 auf, wobei der Abstand der ersten Klemmbacke 13 von der zweiten Klemmbacke 14 durch eine insbesondere manuell betätigbare Schraubvorrichtung 15 veränderbar ist, derart, dass die Klemmbacken aufeinander zu und voneinander wegbewegbar sind. In der dargestellten Ausführungsform ist die zweite Klemmbacke 14 plattenförmig ausgebildet, wobei der wirksame Klemmbereich 140 der zweiten Klemmbacke 14 eine Ausbuchtung aufweist, welche an die Form eines stangen- oder stabförmigen Gegenstandes angepasst ist. Die erste Klemmbacke 13 besteht aus parallel verlaufenden Rundstäben. An einem distalen Ende der Klemmbacke 13 sind die beiden Rundstäbe über eine enge Biegung zur Bildung des wirksamen Klemmbereichs 130 der ersten Klemmbacke 13 miteinander verbunden. Der wirksame Klemmbereich 130 der ersten Klemmbacke 13 ist damit an die Form eines plattenförmigen, ebenen sich flächenhaft erstreckenden Gegenstandes angepasst. Die Klemmvorrichtung 12 ist dazu eingerichtet, an stab- oder plattenförmigen Gegenstanden einer Dicke oder Stärke von 10-50 mm, insbesondere 15-45 mm befestigbar zu sein. An dem proximalen Ende sind die beiden Rundstäbe an dem proximalen Ende der zweiten Klemmbacke 14 angelenkt.

Die hinsichtlich der Klemmwirkung an einem Gegenstand wirksamen Bereiche 130, 140 der Klemmbacken weisen jeweils einen Kunststoffüberzug auf, um die Oberfläche des Gegenstandes zu schonen.

Die zweite Klemmbacke 14 weist eine Bohrung auf, durch die ein unterer Abschnitt eines unten näher beschriebenen Bolzenstifts 63 hindurchgeführt ist. Zuvor ist der Bolzenstift 63 zwischen den Rundstäben der ersten Klemmbacke 13 hindurchgeführt.

Die zweite Klemmbacke 14 ist zugleich Anschlag für das weiter unten noch näher beschriebene Anschlussmittel 60 des Befestigungsmittels 7.

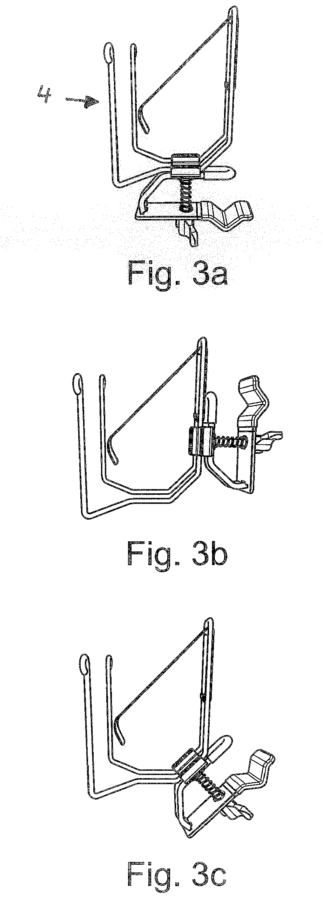

Figuren 3a-3c zeigen den Halter 4 mit dem an den drei verschiedenen Befestigungsbereichen 51, 52, 53 fixierten Befestigungsmittel 7 wie folgt, wobei die Bezugsziffern sich auf die Darstellung der Figuren 1 und 2 beziehen:
Figur 3a entspricht der Darstellung der Figur 1. Das Befestigungsmittel 7 ist an dem ersten Befestigungsbereich 51 fixiert, so dass das Befestigungsmittel 7 eine erste Halteposition einnehmen kann. Der Aufnahmebereich 5 des Halters 4 ist in dieser ersten Halteposition derart ausgerichtet ist, dass die Verpackung in der ersten Halteposition im Wesentlichen liegend aufnehmbar ist. Die erste Halteposition ermöglicht es dem Anwender vorliegend, das Befestigungsmittel 7 an einem sich parallel zu einer horizontalen Ebene im Raum erstreckenden Gegenstand wie beispielsweise einer Stange oder einer Tischkante zu befestigen. Die Gegenstandsebene verläuft also horizontal.

In Figur 3b ist das Befestigungsmittel 7 ist an dem zweiten Befestigungsbereich 52 fixiert, so dass das Befestigungsmittel 7 eine zweite Halteposition einnehmen kann. Der Aufnahmebereich 5 des Halters 4 ist auch in dieser zweiten Halteposition derart ausgerichtet, dass die Verpackung in der zweiten Halteposition im Wesentlichen liegend aufnehmbar ist. Die zweite Halteposition ermöglicht es dem Anwender vorliegend, das Befestigungsmittel 7 an einem sich vertikal im Raum erstreckenden Gegenstand zu befestigen. Die Gegenstandsebene verläuft also vertikal.

In Figur 3c ist das Befestigungsmittel 7 an dem dritten Befestigungsbereich 53 fixiert, so dass das Befestigungsmittel 7 eine dritte Halteposition einnehmen kann. Der Aufnahmebereich 5 des Halters 4 ist mithin auch in diesen Haltepositionen derart ausrichtbar, dass die Verpackung im Wesentlichen liegend aufnehmbar ist. Die dritte Halteposition ermöglichen es dem Anwender vorliegend, das Befestigungsmittel 7 an einem sich schräg im Raum erstreckenden Gegenstand zu befestigen. Die Gegenstandsebene verläuft also in diesen Fällen schräg.

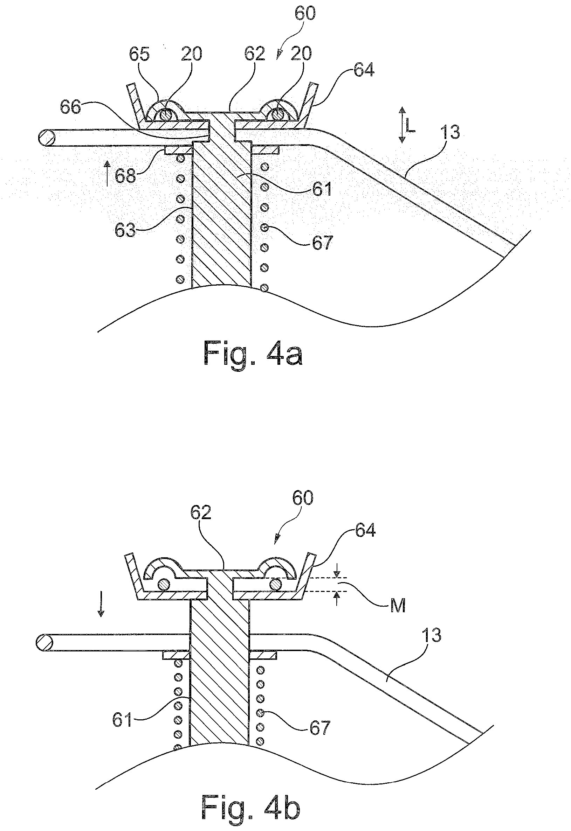

Das Anschlussmittel 60 ist in der nachfolgend mit Bezug zu Figur 4a beschriebenen Weise dafür eingerichtet, die Klemmvorrichtung 12 manuell zwischen jeder der ersten bis dritten Haltepositionen zu bewegen, insbesondere verschieben zu können.

Das Anschlussmittel 60 umfasst einen Bolzen 61 mit einem plattenförmigen Bolzenkopf 62 und einem oben bereits erwähnten Bolzenstift 63. Außerdem eine Anschlagplatte 64, wobei die Anschlagplatte 64 und der Bolzenkopf 62 dafür eingerichtet sind, ein Paar parallel verlaufender Rundstäbe des ersten oder zweiten oder dritten Befestigungsbereichs des Halters zwischen der Anschlagplatte 64 und dem Bolzenkopf 62 unverlierbar aufzunehmen. Hierzu weist der Bolzenkopf 62 zwei entsprechend geformte parallel verlaufende Ausbuchtungen oder Wellen 65 auf. Im dargestellten Fall der Figur 4a sind die einen ersten Befestigungsbereich bildenden Rundstäbe 20 aufgenommen. Die Anschlagplatte 64 besitzt eine Bohrung, durch welche ein oberer, Bolzenkopf - naher, verengter Bolzenstiftabschnitt 66 hindurchgeführt ist. Hierzu weist der Bolzenstift 63 in diesem oberen Abschnitt eine Verringerung seines Durchmessers D auf D1 auf. Die Längserstreckung der Verengung, also deren Erstreckung in Längsrichtung L, ist größer als die Dicke der Anschlagplatte 64. Die Bohrung der Anschlagplatte ist groß genug, um den verengten Bolzenstiftabschnitt 66 aufzunehmen. Sie ist aber klein genug, um gegen den nicht verengten Abschnitt des Bolzenstifts anzuschlagen, so dass die Anschlagplatte 64 mit Spiel in einer Längsrichtung L zwischen dem Bolzenkopf 62 und dem Ende des verengten Bolzenstiftabschnitts 66 unverlierbar gehalten ist. Unterhalb der Anschlagplatte 64 ist der Bolzenstift 63 wie oben beschrieben zwischen den Rundstäben der ersten Klemmbacke 13 und weiter durch eine Bohrung der zweiten Klemmbacke 14 hindurch geführt (in der Teilansicht der Figur 4a nicht dargestellt). Zwischen den Klemmbacken 13, 14 ist über den Bolzenstift 63 eine Spiralfeder 67 gezogen.

Der Bolzenstift 63 ist auch Teil der Schraubvorrichtung 15 (Figur 1). Hierzu weist der Bolzenstift 63 an einem Fußteil ein Außengewinde auf. Die Schraubvorrichtung 15 umfasst außerdem eine Schraubmutter mit Innengewinde, welche auf den Fußteil des Bolzenstifts 63 aufgeschraubt ist. Die Schaubmutter ist im dargestellten Fall als eine manuell betätigbare Flügelmutter 68 ausgebildet. Nach einer alternativen Ausführungsform kann anstelle der Flügelmutter 68 eine mittels separatem Schlüssel abschließbare mit einem Schloss versehene Griffschraube, insbesondere eine Sterngriffschraube, vorgesehen sein.

Die in Längsrichtung L des Bolzenzstifts 63 wirkende Kraft der über den Bolzenstift 63, in einem Abschnitt zwischen den Klemmbacken 13, 14 gezogenen Spiralfeder 67 drückt die erste Klemmbacke 13 gegen die Anschlagplatte 64. In der dargestellten Ausführung ist eine Unterlegscheibe 68 zwischen der Spiralfeder und der ersten Klemmbacke 13 angeordnet, um den Druck der Spiralfeder 67 gleichmäßig auf die erste Klemmbacke 13 übertragen zu können. Durch den über die Spiralfeder 67 auf die Anschlagplatte 64 ausgeübten Druck (in Pfeilrichtung, Figur 4a) liegt die Anschlagplatte 64 unmittelbar an dem Bolzenkopf 62 an, so dass die einen Befestigungsbereich bildenden Rundstäbe - im Falle der Figur 4a die den ersten Befestigungsbereich bildenden Rundstäbe 20 - kraft- und formschlüssig zwischen Anschlagplatte 64 und Bolzen-Kopfteil 62 gehalten sind. Dieser Zustand ist in Figur 4a schematisch skizziert.

Im Falle der Befestigung der Klemmvorrichtung 12 an einem Gegenstand wird der Gegenstand zwischen die hinsichtlich der Klemmwirkung wirksamen Bereiche 130, 140 der Klemmbacken 13, 14 geführt. Anschließend erfolgt das Festschrauben vermittels der Betätigung der Flügelmutter 68, so dass die Klemmbacken 13, 14 zunächst gegen den Widerstand der Spiralfeder und anschließend gegen den Widerstand des eingeklemmten Gegenstandes aufeinander zu bewegt werden. Dabei drückt mit zunehmender Klemmwirkung die erste Klemmbacke 13 mit zunehmender Kraft gegen die Anschlagplatte 64, so dass die den ersten Befestigungsbereich 51 bildenden Rundstäbe zunehmend fester zwischen Anschlagplatte 64 und Bolzen-Kopfteil 62 gehalten sind und fixiert sind. Mithin ist durch die Schraubvorrichtung 15 sowohl eine Befestigung des Halters 4 an den Gegenständen als auch gleichzeitig eine kraftschlüssige Fixierung der Klemmvorrichtung an dem ersten oder an dem zweiten, oder an dem dritten Befestigungsbereich herstellbar.

Es hat sich als besonders vorteilhaft erwiesen, dass der Halter 4 gemeinsam mit dem Anschlussmittel 60 relativ zu den Klemmbacken 13, 14 drehbar, insbesondere wie dargestellt um 360° um eine Längsachse des Bolzenstifts drehbar ist.

Weiterhin hat sich als besonders vorteilhaft erwiesen, dass das Anschlussmittel 64 dafür eingerichtet ist, das Befestigungsmittel 7 manuell zwischen jeder der ersten bis dritten Haltepositionen verschieben zu können. Solange die Klemmvorrichtung 12 nicht an einem Gegenstand befestigt ist, lassen sich die Klemmbacken 13, 14 manuell - z. Bsp. zwischen Daumen und Zeigefinger eines Anwenders und gegen die leichte Federkraft der Spiralfeder aufeinander zu bewegen. Hierdurch wird die Anschlagplatte 64 von dem Druck der Spiralfeder befreit, wodurch die Andruckplatte 64 von dem Bolzenkopf 62 um ein Maß M, im dargestellten Fall um 5,0 mm in Längsrichtung L des Bolzenstifts 61, entlang des verengten Bolzenstiftabschnitts 66 wegbewegbar ist. Dieser Zustand ist in Figur 4b schematisch skizziert. Das Maß M und die Formgebung der Anschlagplatte 64 und des Bolzen-Kopfteils 62 sind so aufeinander abgestimmt und dafür eingerichtet, dass die den ersten, zweiten und/oder dritten Befestigungsbereich bildenden Rundstäbe auch in diesem Zustand maximal möglichen Abstandes zwischen Anschlagplatte 64 und Bolzen-Kopfteils 62 unverlierbar gehalten sind. Hingegen lässt sich in diesem Zustand die Klemmvorrichtung 12 mühelos von einer Halteposition in eine andere verschieben. Hierzu sind das Maß M und die Formgebung und Abmessung der Anschlagplatte 64 und des Bolzen-Kopfteils 62 so aufeinander abgestimmt und dafür eingerichtet, dass das Anschlussmittel 60 auch über die kurzen Bögen, welche die Befestigungsbereiche voneinander trennen, hinwegbewegbar ist, so dass die in Figuren 3a-3c dargestellten ersten bis dritten Haltepositionen einnehmbar sind.

Hierdurch ist dem Anwender in besonders komfortabler Weise somit möglich, verschiedene Haltepositionen der Klemmvorrichtung vorzusehen, um die am Point-of-Care konkret sich darbietenden Gegenstände flexibel zur Befestigung des Halters nutzen zu können.

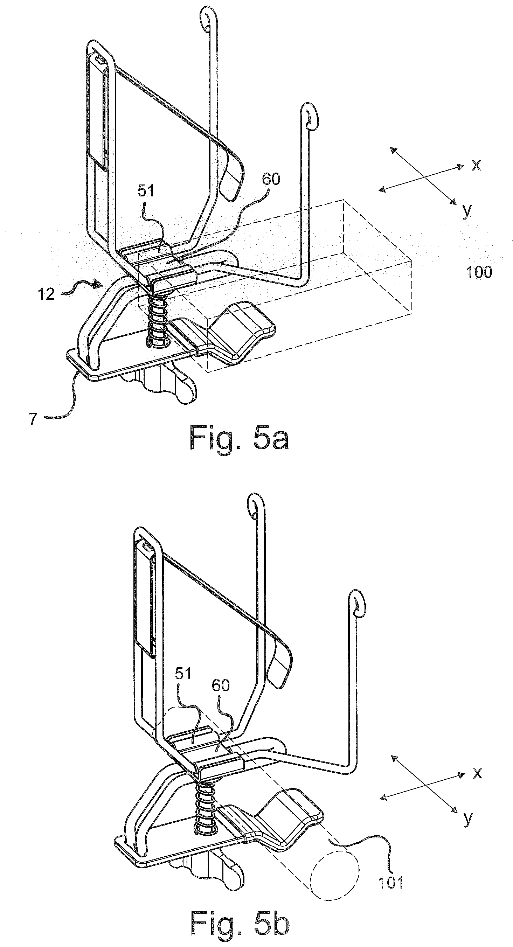

In der dargestellten Ausführungsform des Haltesystems 1 ist die Befestigungsbereichsebene, also die Ebene, in welcher das Paar die jeweiligen Befestigungsbereiche des Halters bildenden Rundstäbe in einer Halteposition verläuft, parallel zu einer Gegenstandsebene orientiert, also zu einer Ebene orientiert, in der die Gegenstände verlaufen, an welche in einer jeweiligen Halteposition die Klemmvorrichtung befestigbar ist. Beispielhaft zeigen dies Figuren 5a und 5b. Hier ist die das Befestigungsmittel 7 bildende Klemmvorrichtung 12 in der ersten Halteposition an dem ersten Befestigungsbereich 51 fixiert. In der Anwendungssituation verlaufen die den ersten Befestigungsbereich bildenden Rundstäbe (in der Ansicht der Figuren 5a und 5b durch das Anschlussmittel 60 verdeckt, also nicht sichtbar) in einer horizontal verlaufenden Ebene, um eine Verpackung in den Aufnahmebereich des Halters aufnehmen zu können. Die hinsichtlich der Klemmwirkung wirksamen Bereiche der Klemmbacken sind dabei so ausgerichtet, dass sie an Gegenständen fixierbar sind, welche ebenfalls parallel zu einer horizontal verlaufenden Ebene verlaufen. Im Falle der Figur 5a ist dieser Gegenstand schematisch als ein plattenförmiger Abschnitt einer Tischkante 100 dargestellt. In Figur 5b ist der Gegenstand schematisch als ein Abschnitt einer Bettstange 101 dargestellt. In den Figuren 5a und 5b ist eine horizontal verlaufende Ebene durch die sich kreuzenden Pfeile X und Y angedeutet. In beiden Fällen verläuft die Gegenstandsebene parallel zur Befestigungsebene.

Die Parallelität von Befestigungsebene und jeweiliger Gegenstandsebene hat den Vorteil, dass der Anwender, welcher beabsichtigt, das Haltesystem am Point-of-Care an einem Gegenstand zu befestigen, sehr schnell die Entscheidung für eine geeignete Halteposition, mithin den für die Fixierung des Befestigungsmittels 7 am Halter am besten geeigneten Befestigungsbereich treffen kann.

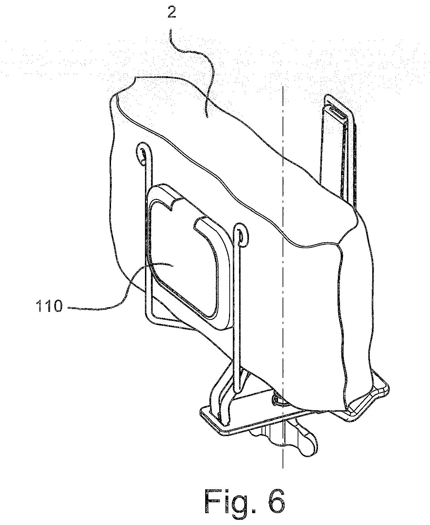

Figur 6 veranschaulicht, wie eine Verpackung 2, beispielsweise eine Verpackung mit Desinfektionstüchern, in einer Ausführungsform des erfindungsgemäßen Haltesystems 1 gehalten wird. Die im Wesentlichen quaderförmige Verpackung 2, die im gezeigten Beispiel als Folienbeutel ausgebildet ist, ist liegend im Aufnahmebereich 5 eingeklemmt. Eine Seitenfläche liegt hierbei auf dem Boden 8 (siehe Figur 1) des Haltesystems 1, während die andere Seitenfläche aus dem Aufnahmebereich 5 herausragt. Die Deckfläche, an welcher die Entnahmeöffnung 110 vorhanden ist, ist zum Vorderteil 10 des Haltesystems 1 hin ausgerichtet. Die Entnahmeöffnung 110 ist hierbei zwischen den Rundstäben 19 angeordnet und für einen Nutzer frei zugänglich. Die Grundfläche der Verpackung 2 zeigt zum Rückteil 9 des Haltesystems 1, an welchem das Federelement 103 mit Befestigungskomponente 104 und Blattfeder 105 befestigt ist. Die Blattfeder 105 drückt mit ihrem freien Ende im Wesentlichen mittig gegen die Grundfläche der Verpackung 2 und kann hierbei die in der Verpackung 2 enthaltenen Artikel in Richtung der Entnahmeöffnung 110 schieben.

Bei der Darstellung in Figur 6 ist das Befestigungsmittel 7 derart orientiert, dass die Klemmvorrichtung 12 beispielsweise an einer horizontalen Stange oder Fläche angebracht sein könnte (siehe Figur 5). Hierbei ist das Befestigungsmittel 7 an dem ersten Befestigungsbereich 51 fixiert. Diese hier nur beispielhaft gezeigte Anordnung führt dazu, dass die Deckfläche der Verpackung 2 mit Entnahmeöffnung 110 zur Seite hin zeigt. Eine derartige Anordnung ist in einer Gebrauchssituation insbesondere bei der Bereitstellung von Reinigungs- oder Desinfektionstüchern vorteilhaft, da die in der Verpackung 2 vorhandene Reinigungs- oder Desinfektionsflüssigkeit nicht zum Boden der Verpackung 2 sickert. Somit ist eine gleichmäßigere Befeuchtung der in der Verpackung 2 enthaltenen Tücher gewährleistet. Es wäre jedoch auch möglich, das Befestigungsmittel 7 an den zweiten Befestigungsbereich 52 zu fixieren. In diesem Falle würde dann die Entnahmeöffnung 110 der Verpackung 2 nach oben zeigen. Dies könnte insbesondere bei der Bereitstellung von Einmal-Handschuhen vorteilhaft sein, da die Handschuhe ein besonders einfach herausgenommen werden können.

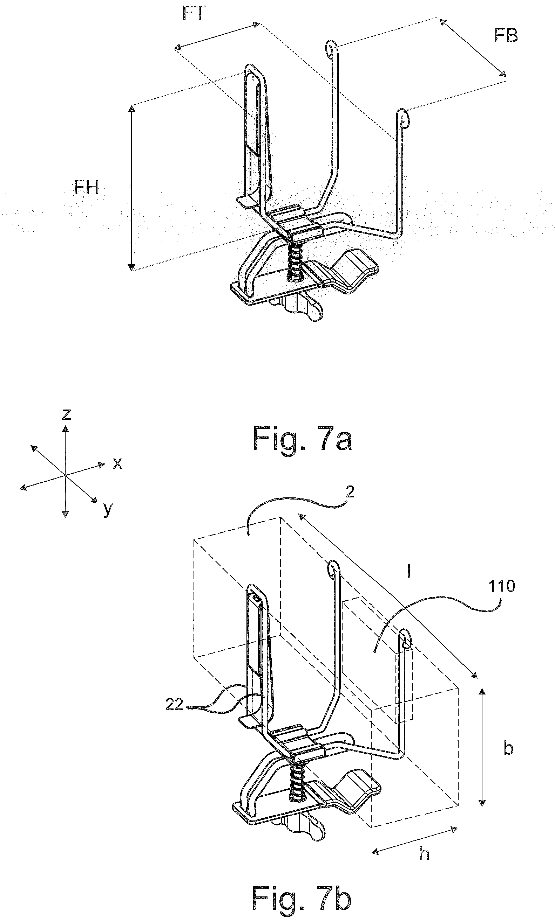

Figur 7b skizziert schematisch als punktierte Linie eine Verpackung 2, welche in den Aufnahmebereich eines erfindungsgemäßen Haltesystems aufgenommen ist. Die Verpackung 2 besitzt beispielhaft die Form eines Quaders der Länge I, der Höhe h und der Breite b. Die Verpackung liegt mit ihrer Seite auf dem Boden 8 des Halters, so dass die Deckfläche und die Grundfläche des Quaders (b x I) vertikal sind und die langen Seitenflächen (h x I) horizontal ausgerichtet sind. Die Länge I beträgt 250 mm. Die Höhe h beträgt 80 mm. Die Breite b beträgt 100 mm. Die Verpackung 2 weist eine Entnahmeöffnung 110 auf.

Skizziert sind außerdem in Figur 7a die Höhe FH, die Breite FB und die Tiefe FT des Aufnahmebereichs des Halters. Die Tiefe FT beträgt 95 mm. Hierbei wird das Verständnis unterstellt, dass die Tiefe FT des Aufnahmebereichs durch den Abstand der die Tiefe des Aufnahmebereichs begrenzenden Bauteile des Halters in x-Richtung definiert ist. Die Höhe FH des Aufnahmebereichs beträgt 95 mm und entspricht der maximalen Erstreckung der den Aufnahmebereich bildenden Bauteile des Halters in z-Richtung. Die Breite FB des Aufnahmebereichs ist durch den Abstand der die Breite des Aufnahmebereichs begrenzenden Bauteile des Halters in y-Richtung definiert. Im dargestellten Fall ist dies der Abstand der beiden Stützelemente des Vorderteils des Halters voneinander, welcher im dargestellten Fall FB= 80 mm beträgt.

Erkennbar ist außerdem, dass nur ein äußerst geringer Flächenanteil der vier senkrecht orientierten Sichtseiten der Verpackung 2 durch den Halter verdeckt ist. Hierdurch hat der Anwender die Gelegenheit, eventuelle auf den Sichtflächen vorhandene Informationen wie bedruckte Etiketten, welche auf die Sichtseiten aufgebracht sind, wahrnehmen zu können. Im dargestellten Fall sind etwa lediglich 5% einer jeweiligen Fläche durch die Rundstäbe des Halters abgedeckt, so dass etwa 95% der jeweiligen Fläche durch den Anwender frei betracht- und einsehbar ist. Erkennbar ist auch, dass die Entnahmeöffnung 110 zwischen den Rundstäben 19 für einen Nutzer frei zugänglich ist.

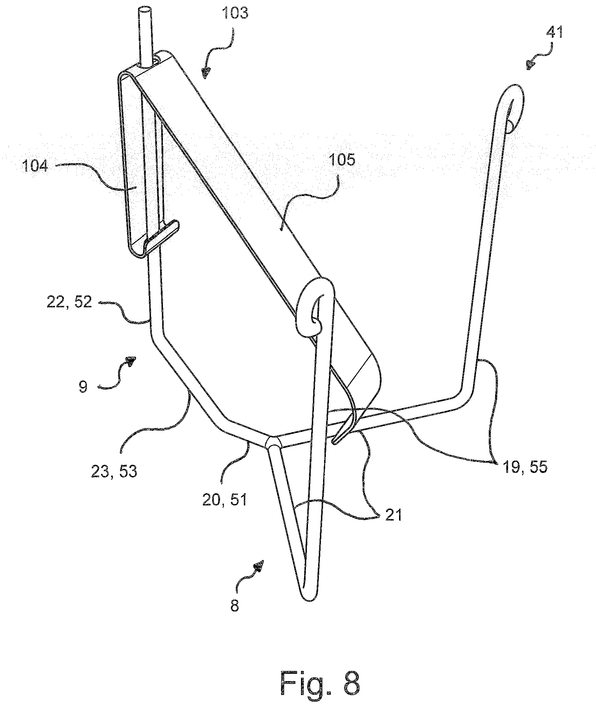

Figur 8 zeigt einen weiteren Halter 41 eines erfindungsgemäßen Haltesystems, bei dem ein erster Abschnitt des Bodens 8 des Halters 41 durch einen einzelnen Rundstab 20 zur Bildung des ersten Befestigungsbereichs 51 ausgebildet ist. Auch das Rückteil 9 ist durch einen einzelnen Rundstab 22 zur Bildung des zweiten Befestigungsbereichs 52 ausgebildet. Schließlich ist der dritte Befestigungsbereich 53 durch einen einzelnen Rundstab 23 des Zwischenteils des Halters ausgebildet. Der zweite Abschnitt des Bodens 8 hingegen ist durch ein Paar Rundstäbe 21 gebildet, welche sich ausgehend von dem Ende des Rundstabs 20 des ersten Abschnitts des Bodens 8 V-artig mit größer werdendem Abstand erstrecken, um über einen kurzen Bogen in Rundstäbe 19 überzugehen, welche die vorderen Stützelemente 55 des Halters 41 bilden. Die Befestigungskomponente 104 des Federelements 103 ist mittels zwei Bohrungen am Rundstab 22 fixiert.

Ein nicht dargestelltes Befestigungsmittel kann beispielsweise eine wie mit Bezug zu Figur 1 beschriebene Klemmvorrichtung aufweisen. Ein Anschlussmittel zum Anschließen der Klemmvorrichtung an den Halter 41 kann solchen Falls an die Form eines einzelnen Rundstabes angepasst sein, so dass das Befestigungsmittel vermittels des Anschlussmittels sowohl an dem ersten Befestigungsbereich, als auch an dem zweiten Befestigungsbereich als auch an dem dritten Befestigungsbereich anschließbar ist und über die Befestigungsbereiche hinwegbewegbar ist.

## Patentansprüche

1. Haltesystem (1) zum Bereitstellen einer insbesondere mit Reinigungs- oder Desinfektionstüchern oder mit Einmal-Handschuhen versehenen Verpackung (2), wobei die Verpackung eine Entnahmeöffnung aufweist, umfassend:
einen Halter (4, 41) mit einem Aufnahmebereich (5) für die Verpackung (2), wobei der Halter (4, 41) einen ersten Befestigungsbereich (51), einen zweiten Befestigungsbereich (52) und einen dritten Befestigungsbereich (53) aufweist, wobei der erste (51), zweite (52) und dritte Befestigungsbereich (53) Drähte oder Stäbe umfassen, und wobei die Drähte oder Stäbe des ersten (51), zweiten (52) oder dritten Befestigungsbereichs (53) Abschnitte des Aufnahmebereichs des Halters (4, 41) bilden, und wobei die Drähte oder Stäbe des ersten Befestigungsbereichs (51) mindestens einen Abschnitt eines Bodens (8) des Halters (4, 41) bilden, und wobei die Drähte oder Stäbe des zweiten Befestigungsbereichs (52) mindestens einen Abschnitt eines Rückteils (9) des Halters (4, 41) bilden,
ein Befestigungsmittel (7) zur lösbaren Befestigung des Halters (4, 41) an Gegenständen, wobei das Befestigungsmittel (7) an dem ersten Befestigungsbereich (51) fixierbar ist, und das Befestigungsmittel (7) dadurch eine erste Halteposition einnehmen kann, und das Befestigungsmittel (7) an dem zweiten Befestigungsbereich (52) fixierbar ist, und das Befestigungsmittel (7) dadurch eine zweite Halteposition einnehmen kann, und das Befestigungsmittel (7) an dem dritten Befestigungsbereich (53) fixierbar ist, und das Befestigungsmittel (7) dadurch eine dritte Halteposition einnehmen kann, und ein an dem Halter vorhandenes Federelement (103), welches eine Kraft auf die in den Aufnahmebereich eingelegte Verpackung (2) ausüben kann, so dass die Verpackung (2) in dem Aufnahmebereich (5) gehalten wird, wobei das Federelement eine Blattfeder umfasst,
wobei dassdie Blattfeder (105) in einem ungespannten Zustand in den Aufnahmebereich (5) hineinragt und beim Einlegen der Verpackung (2) in den Aufnahmebereich (5) zum Rückteil (9) des Halters hin wegbewegt wird.

2. Haltesystem (1) nach Anspruch 1, **dadurch gekennzeichnet, dass** es sich bei der Verpackung um eine Flowpack-Verpackung handelt, welche insbesondere ein Verschlusselement aufweist.

3. Haltesystem (1) nach Anspruch 1 oder 2, **dadurch gekennzeichnet, dass** das Federelement (103) eine Kraft auf die der Entnahmeöffnung (110) gegenüberliegende Seite der in den Aufnahmebereich (5) eingelegten Verpackung (2) ausüben kann.

4. Haltesystem (1) nach einem oder mehreren der Ansprüche 1 bis 3, **dadurch gekennzeichnet, dass** die erste Halteposition die Befestigung des Halters (4, 41) an einem horizontal orientierten Gegenstand ermöglicht und die zweite Halteposition die Befestigung des Halters (4, 41) an einem senkrecht orientierten Gegenstand ermöglicht und die dritte Halteposition die Befestigung des Halters (4, 41) an einem schräg orientierten Gegenstand ermöglicht.

5. Haltesystem (1) nach einem oder mehreren der Ansprüche 1 bis 4, **dadurch gekennzeichnet, dass** das Federelement (103) an einen Abschnitt eines Rückteils (9) des Halters (4, 41) unlösbar befestigt ist.

6. Haltesystem (1) nach einem oder mehreren der Ansprüche 1 bis 5, **dadurch gekennzeichnet, dass** die Blattfeder (105) in dem durch das Einlegen der Verpackung (2) in den Aufnahmebereich (5) sich ergebenden gespannten Zustand eine im Wesentlichen mittig auf die der Entnahmeöffnung (110) gegenüberliegende Rückseite der Verpackung (2) wirkende Kraft ausübt.

7. Haltesystem (1) nach einem oder mehreren der Ansprüche 1 bis 6, **dadurch gekennzeichnet, dass** die Drähte oder Stäbe des dritten Befestigungsbereichs (53) zwischen dem ersten (51) und zweiten (52) Befestigungsbereich angeordnet sind.

8. Haltesystem (1) nach einem oder mehreren der Ansprüche 1 bis 7, **dadurch gekennzeichnet, dass** der Halter (4, 41) vordere Stützelemente (55) aufweist, welche einen Abschnitt eines Vorderteils (10) des Halters (4, 41) bilden, wobei die vorderen Stützelemente (55) die Entnahmeöffnung (110) der in den Aufnahmebereich eingelegten Verpackung (2) umgreifen, so dass die Entnahmeöffnung (110) in einer Anwendungssituation für einen Nutzer frei zugänglich ist.

9. Haltesystem (1) nach einem oder mehreren der Ansprüche 1 bis 8, **dadurch gekennzeichnet, dass** die Drähte oder Stäbe des zweiten Befestigungsbereichs (52) senkrecht zu den Drähten oder Stäben des ersten Befestigungsbereichs (51) verlaufen.

10. Haltesystem (1) nach einem oder mehreren der Ansprüche 1 bis 9, **dadurch gekennzeichnet, dass** die Drähte oder Stäbe des dritten Befestigungsbereichs (53) schräg zu den Drähten oder Stäben des ersten Befestigungsbereichs (51) verlaufen, insbesondere in einem Winkel von 10° bis 80°, weiter insbesondere von 20° bis 60°, weiter insbesondere von 30 bis 50°.

11. Haltesystem (1) nach einem oder mehreren der Ansprüche 1 bis 10, **dadurch gekennzeichnet, dass** der Halter (4, 41) durch einen durchgehenden, insbesondere abschnittsweise gebogenen Draht oder Stab gebildet ist.

12. Haltesystem (1) nach einem oder mehreren der Ansprüche 1 bis 11, **dadurch gekennzeichnet, dass** das Befestigungsmittel (7) eine Klemmvorrichtung (12) aufweist, vermittels derer die Befestigung des Halters (4, 41) an den Gegenständen herstellbar ist, und weiter **dadurch gekennzeichnet, dass** die Klemmvorrichtung (12) eine erste Klemmbacke (13) und eine zweite Klemmbacke (14) aufweist, wobei der Abstand der ersten Klemmbacke (13) von der zweiten Klemmbacke (14) veränderbar ist.

13. Haltesystem (1) nach einem oder mehreren der Ansprüche 1 bis 12, **dadurch gekennzeichnet, dass** das Befestigungsmittel (7) ein Anschlussmittel (60) aufweist und das Anschlussmittel (60) dafür eingerichtet ist, das Befestigungsmittel insbesondere manuell zwischen jeder der ersten bis dritten Haltepositionen verschieben zu können.

14. Haltesystem (1) nach einem oder mehreren der Ansprüche 1 bis 13, **dadurch gekennzeichnet, dass** der Halter (4, 41) relativ zu dem Befestigungsmittel (7) um eine Achse um vorzugsweise 360° drehbar, insbesondere stufenlos drehbar gelagert ist.

15. System umfassend ein Haltesystem (1) nach einer oder mehreren der Ansprüche 1 bis 14 und eine Verpackung, insbesondere mit Reinigungs- oder Desinfektionstüchern oder mit Einmal-Handschuhen, wobei die Verpackung (2) in den Aufnahmebereich (5) einbringbar ist.

## Claims

1. Holding system (1) for making available a package (2) provided in particular with cleaning or disinfectant wipes or with disposable gloves, wherein the package has a removal opening, comprising:
a holder (4, 41) with a receiving zone (5) for the package (2), wherein the holder (4, 41) has a first fastening zone (51), a second fastening zone (52) and a third fastening zone (53), wherein the first (51), second (52) and third fastening zones (53) comprise wires or rods, and wherein the wires or rods of the first (51), second (52) or third fastening zone (53) form portions of the receiving zone of the holder (4, 41), and wherein the wires or rods of the first fastening zone (51) form at least a portion of a bottom (8) of the holder (4, 41), and wherein the wires or rods of the second fastening zone (52) form at least a portion of a rear part (9) of the holder (4, 41),
a fastening means (7) for releasable fastening the holder (4, 41) to objects, wherein the fastening means (7) is able to be fixed in the first fastening zone (51), with the result that the fastening means (7) can take up a first holding position, and the fastening means (7) is able to be fixed in the second fastening zone (52), with the result that the fastening means (7) can take up a second holding position, and the fastening means (7) is able to be fixed in the third fastening zone (53), with the result that the fastening means (7) can take up a third holding position, and a spring element (103) which is present on the holder and can exert a force on the package (2) introduced into the receiving zone such that the package (2) is held in the receiving zone (5), wherein the spring element comprises a leaf spring,
wherein the leaf spring (105) projects into the receiving zone (5) in an untensioned state and is moved away towards the rear part (9) of the holder when the package (2) is introduced into the receiving zone (5).

2. Holding system (1) according to Claim 1, **characterized in that** the package is a flowpack package, which has in particular a closure element.

3. Holding system (1) according to Claim 1 or 2, **characterized in that** the spring element (103) can exert a force on the opposite side of the package (2) introduced into the receiving zone (5) from the removal opening (110).

4. Holding system (1) according to one or more of Claims 1 to 3, **characterized in that** the first holding position allows the holder (4, 41) to be fastened to a horizontally oriented object and the second holding position allows the holder (4, 41) to be fastened to a vertically oriented object and the third holding position allows the holder (4, 41) to be fastened to a obliquely oriented object.

5. Holding system (1) according to one or more of Claims 1 to 4, **characterized in that** the spring element (103) is non-releasably fastened to a portion of a rear part (9) of the holder (4, 41).

6. Holding system (1) according to one or more of Claims 1 to 5, **characterized in that**, in the tensioned state that arises from the introduction of the package (2) into the receiving zone (5), the leaf spring (105) exerts a force acting substantially centrally on the rear side of the package (2), which is on the opposite side from the removal opening (110).

7. Holding system (1) according to one or more of Claims 1 to 6, **characterized in that** the wires or rods of the third fastening zone (53) are arranged between the first (51) and second (52) fastening zones.

8. Holding system (1) according to one or more of Claims 1 to 7, **characterized in that** the holder (4, 41) has front support elements (55) which form a portion of a front part (10) of the holder (4, 41), wherein the front support elements (55) engage around the removal opening (110) of the package (2) introduced into the receiving zone, such that the removal opening (110) is freely accessible to a user in a use situation.

9. Holding system (1) according to one or more of Claims 1 to 8, **characterized in that** the wires or rods of the second fastening zone (52) extend perpendicularly to the wires or rods of the first fastening zone (51).

10. Holding system (1) according to one or more of Claims 1 to 9, **characterized in that** the wires or rods of the third fastening zone (53) extend obliquely to the wires or rods of the first fastening zone (51), in particular at an angle of 10° to 80°, more particularly 20° to 60°, more particularly 30° to 50°.

11. Holding system (1) according to one or more of Claims 1 to 10, **characterized in that** the holder (4, 41) is formed by a continuous wire or rod that is in particular bent in certain sections.

12. Holding system (1) according to one or more of Claims 1 to 11, **characterized in that** the fastening means (7) has a clamping device (12), by means of which the fastening of the holder (4, 41) to the objects is able to be established, and also **characterized in that** the clamping device (12) has a first clamping jaw (13) and a second clamping jaw (14), wherein the distance of the first clamping jaw (13) from the second clamping jaw (14) is variable.

13. Holding system (1) according to one or more of Claims 1 to 12, **characterized in that** the fastening means (7) has a connection means (60) and the connection means (60) is designed for it to be possible to shift the fastening means, in particular manually, between each of the first to third holding positions.

14. Holding system (1) according to one or more of Claims 1 to 13, **characterized in that** the holder (4, 41) is mounted so as to be rotatable, in particular infinitely rotatable, through preferably 360° about an axis relative to the fastening means (7).

15. System comprising a holding system (1) according to one or more of Claims 1 to 14 and a package, in particular with cleaning or disinfectant wipes or with disposable gloves, wherein the package (2) is able to be introduced into the receiving zone (5).

## Revendications

1. Système de support (1) pour fournir un emballage (2), notamment muni de lingettes nettoyantes ou désinfectantes ou de gants jetables, l'emballage présentant une ouverture de prélèvement, comprenant :
un support (4, 41) avec une zone de réception (5) pour l'emballage (2), le support (4, 41) présentant une première zone de fixation (51), une deuxième zone de fixation (52) et une troisième zone de fixation (53), la première (51), la deuxième (52) et la troisième (53) zone de fixation comprenant des fils ou des tiges, et les fils ou les tiges de la première (51), de la deuxième (52) ou de la troisième (53) zone de fixation formant des sections de la zone de réception du support (4, 41), et les fils ou les tiges de la première zone de fixation (51) formant au moins une section d'un fond (8) du support (4, 41), et les fils ou les tiges de la deuxième zone de fixation (52) formant au moins une section d'une partie arrière (9) du support (4, 41),
un moyen de fixation (7) pour fixer de manière amovible le support (4, 41) sur des objets, le moyen de fixation (7) pouvant être fixé sur la première zone de fixation (51), et le moyen de fixation (7) pouvant ainsi prendre une première position de support, et le moyen de fixation (7) pouvant être fixé sur la deuxième zone de fixation (52), et le moyen de fixation (7) pouvant ainsi prendre une deuxième position de support, et le moyen de fixation (7) pouvant être fixé sur la troisième zone de fixation (53), et le moyen de fixation (7) pouvant ainsi prendre une troisième position de support, et un élément de ressort (103) présent sur le support, qui peut exercer une force sur l'emballage (2) inséré dans la zone de réception, de telle sorte que l'emballage (2) est supporté dans la zone de réception (5), l'élément de ressort comprenant un ressort à lame,
le ressort à lame (105) avançant dans la zone de réception (5) dans un état non tendu et s'éloignant de la partie arrière (9) du support lors de l'insertion de l'emballage (2) dans la zone de réception (5).

2. Système de support (1) selon la revendication 1, **caractérisé en ce que** l'emballage consiste en un emballage Flowpack, qui présente notamment un élément de fermeture.

3. Système de support (1) selon la revendication 1 ou 2, **caractérisé en ce que** l'élément de ressort (103) peut exercer une force sur le côté de l'emballage (2) inséré dans la zone de réception (5) qui est opposé à l'ouverture de prélèvement (110).

4. Système de support (1) selon une ou plusieurs des revendications 1 à 3, **caractérisé en ce que** la première position de support permet de fixer le support (4, 41) sur un objet orienté horizontalement, et la deuxième position de support permet de fixer le support (4, 41) sur un objet orienté verticalement, et la troisième position de support permet de fixer le support (4, 41) sur un objet orienté en oblique.

5. Système de support (1) selon une ou plusieurs des revendications 1 à 4, **caractérisé en ce que** l'élément de ressort (103) est fixé de manière inamovible sur une section d'une partie arrière (9) du support (4, 41).

6. Système de support (1) selon une ou plusieurs des revendications 1 à 5, **caractérisé en ce que** le ressort à lame (105), dans l'état tendu résultant de l'insertion de l'emballage (2) dans la zone de réception (5), exerce une force agissant essentiellement au centre sur le côté arrière de l'emballage (2) qui est opposé à l'ouverture de prélèvement (110).

7. Système de support (1) selon une ou plusieurs des revendications 1 à 6, **caractérisé en ce que** les fils ou les tiges de la troisième zone de fixation (53) sont agencés entre la première (51) et la deuxième (52) zone de fixation.

8. Système de support (1) selon une ou plusieurs des revendications 1 à 7, **caractérisé en ce que** le support (4, 41) présente des éléments de soutien avant (55) qui forment une section d'une partie avant (10) du support (4, 41), les éléments de soutien avant (55) entourant l'ouverture de prélèvement (110) de l'emballage (2) inséré dans la zone de réception (35), de telle sorte que l'ouverture de prélèvement (110) est librement accessible à un utilisateur dans une situation d'utilisation.

9. Système de support (1) selon une ou plusieurs des revendications 1 à 8, **caractérisé en ce que** les fils ou les tiges de la deuxième zone de fixation (52) s'étendent perpendiculairement aux fils ou aux tiges de la première zone de fixation (51).

10. Système de support (1) selon une ou plusieurs des revendications 1 à 9, **caractérisé en ce que** les fils ou les tiges de la troisième zone de fixation (53) s'étendent en oblique par rapport aux fils ou aux tiges de la première zone de fixation (51), notamment selon un angle de 10° à 80°, plus particulièrement de 20° à 60°, plus particulièrement de 30 à 50°.

11. Système de support (1) selon une ou plusieurs des revendications 1 à 10, **caractérisé en ce que** le support (4, 41) est formé par un fil ou une tige continu (e), notamment courbé(e) par sections.

12. Système de support (1) selon une ou plusieurs des revendications 1 à 11, **caractérisé en ce que** le moyen de fixation (7) présente un dispositif de serrage (12) au moyen duquel la fixation du support (4, 41) sur les objets peut être réalisée, et **caractérisé en outre en ce que** le dispositif de serrage (12) présente une première mâchoire de serrage (13) et une deuxième mâchoire de serrage (14), la distance entre la première mâchoire de serrage (13) et la deuxième mâchoire de serrage (14) étant variable.

13. Système de support (1) selon une ou plusieurs des revendications 1 à 12, **caractérisé en ce que** le moyen de fixation (7) présente un moyen de raccordement (60) et le moyen de raccordement (60) est conçu pour pouvoir déplacer le moyen de fixation, notamment manuellement, entre chacune des première à troisième positions de support.

14. Système de support (1) selon une ou plusieurs des revendications 1 à 13, **caractérisé en ce que** le support (4, 41) est monté de manière à pouvoir tourner par rapport au moyen de fixation (7) autour d'un axe de préférence de 360°, notamment de manière continue.

15. Système comprenant un système de support (1) selon une ou plusieurs des revendications 1 à 14 et un emballage, notamment avec des lingettes nettoyantes ou désinfectantes ou des gants jetables, l'emballage (2) pouvant être introduit dans la zone de réception (5).
